(19)

Europäisches
Patentamt
European
Patent Office
Office européen
des brevets

(11) **EP 2 371 802 A1**

(12) **EUROPEAN PATENT APPLICATION**

(43) Date of publication:
05.10.2011 Bulletin 2011/40

(21) Application number: **10158468.8**

(22) Date of filing: **30.03.2010**

(51) Int Cl.:
*C07C 51/43* (2006.01)          *C07C 51/487* (2006.01)
*C07C 55/10* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicants:
• **DSM IP Assets B.V.**
  **6411 TE Heerlen (NL)**
• **Roquette Frères SA**
  **62136 Lestrem (FR)**

(72) Inventors:
• **Filey, Guillaume**
  **59800 Lille (FR)**
• **Geurts, Theodorus, Gerardus, Elisabeth**
  **Delft (NL)**

(74) Representative: **Cazemier, Anne Engeline et al**
  **DSM Intellectual Property**
  **P.O. Box 130**
  **6100 AC Echt (NL)**

(54) **Process for the crystallization of succinic acid**

(57)    The present invention relates to a process for crystallizing succinic acid from an aqueous solution comprising treating the aqueous solution with an oxidizing agent and crystallizing the succinic acid to form succinic acid crystals.

EP 2 371 802 A1

**Description**

The present invention relates to a process for the crystallization of succinic acid.

Succinic acid is a C4-dicarboxylic acid and is an intermediate of the tricarboxylic acid cycle (TCA). Succinic acid finds numerous applications in the cosmetic, pharmaceutical, and food industry and as a monomer in the preparation of polyester polymers. Thus far, succinic acid is predominantly produced via petrochemical processes which are considered harmful to the environment. The biological production of succinic acid via fermentation has been focussed on as an attractive alternative to petrochemical-based processes. Various bacteria are known to produce succinic acid by fermentation such as *Actinobacillus succinogenes, Mannheimia succiniciproducens* and *Escherichia coli,* as well as fungi such as *Aspergillus niger* and *Saccharomyces cerevisiae.* When succinic acid is produced through microbial fermentation numerous impurities like organic acids, proteins and the like are also produced. These and other impurities need to be removed upon recovery of succinic acid from the fermentation broth to obtain succinic acid crystals with low coloring and a sufficiently high purity.

Several processes for the purification of succinic acid from a fermentation broth have been developed.

US 5,168,055 discloses a process for the recovery of succinic acid from a fermentation broth containing calcium succinate, wherein sulphuric acid was used to convert succinate into succinic acid, and wherein calcium sulphate is produced. The succinic acid was further purified with a strong acidic acid cation exchange resin and a weak basic anion exchange resin. However, these purification steps, do not remove final coloring in the succinic acid crystals sufficiently.

US 6,265,190 discloses a process for the recovery of succinic acid from a fermentation medium comprising succinate, by the addition of ammonium sulphate. The succinic acid crystals were further purified by dissolution in methanol. The use of methanol for purifying succinic acid crystals is undesirable because of its toxicity and remaining traces of methanol in the succinic acid crystals in various applications are undesirable.

WO2009/082050 discloses a method for purifying succinic acid from a microbial culture broth, wherein prior to concentrating and acidifying the culture broth with hydrochloride and sulphuric acid, the culture broth is decolorized with activated carbon.

A disadvantage of decoloring the whole culture broth is that a large amount of activated carbon is needed, which is expensive and not economical.

The aim of the present invention is an alternative process for the recovery of succinic acid at a sufficiently high yield and purity.

The aim is achieved according to the present invention with a process for crystallizing succinic acid from an aqueous solution comprising treating the aqueous solution with an oxidizing agent and crystallizing the succinic acid to form succinic acid crystals.

It was surprisingly found that the succinic acid crystals formed in the process according to the present invention had a reduced content of impurities such as fumaric acid, orotic acid and coloration without a decrease in yield of succinic acid as compared to succinic acid crystals obtained from a process for the crystallization of succinic acid from an aqueous solution wherein the aqueous solution was not treated with an oxidizing agent.

It was found essential to reduce the content of orotic acid and/or fumaric acid in the aqueous solution for a good succinic acid crystal quality, as these acids have a low solubility and may co-crystallize with succinic acid..

An advantage of succinic acid crystals obtained by the process for crystallizing succinic acid according to the present invention was that it appeared to have good properties in applications such as in polyester polymers (polybutylene succinic acid (PBS)), resulting in polyester polymers with low coloration.

Crystallizing succinic acid in the process according to the present invention may be carried out by any method known by the skilled person in the art. Preferably, crystallizing succinic acid from an aqueous solution in the process according to the present invention comprises bringing the temperature of the aqueous to a temperature of between 1 and 25 degrees Celsius, and/or evaporative crystallization. Preferably, crystallizing succinic acid in the process according to the present invention comprises bringing the aqueous solution to a temperature of between 1 and 25 degrees Celsius, preferably between 2 and 20 degrees Celsius.

Preferably, a step of bringing the aqueous solution to a temperature of between 50 and 90 degrees Celsius, preferably between 60 and 80 degrees Celsius, more preferably between 65 and 75 degrees Celsius precedes a step of bringing the aqueous solution to a temperature of between 1 and 25 degrees Celsius,

The step of bringing the aqueous solution to a temperature of between 50 and 100 degrees Celsius is advantageous since this resulted in an aqueous with a higher concentration of succinic acid.

In one preferred embodiment, crystallizing succinic acid in the process according to the present invention comprises a step of bringing the aqueous solution to a pH of between 1 and 4, preferably between 1.5 and 3.5, preferably between 2 and 3 by any suitable method known in the art. Preferably, bringing an aqueous solution to a preferred pH value may be carried out by subjecting the aqueous solution to water-dissociation bipolar electrodialysis. Preferably, subjecting an aqueous solution, for instance a fermentation broth, to water-dissociation bipolar electrodialysis is preceded by a step of treating the aqueous solution with a weak cation exchange resin, such as a diacetic acid or aminophosphoric chelating agent. This latter step was found advantageous to remove divalent cations, in particular $Mg^{2+}$ and $Ca^{2+}$ ions, which are harmful for the membranes of the bipolar electrodialyser. In the event the aqueous solution is a fermentation broth, a step of removing organic components from the fermentation broth preferably precedes subjecting the fermentation broth to water-dissociation bipolar electrodialysis, in order to avoid pollution of the electrodialysis membrane.

A step of subjecting the aqueous solution, such as a fermentation broth, to water-dissociation bipolar electrodialysis usually further comprises a subsequent step of treating the aqueous solution with a strong cation exchange resin such as a polystyrene divinylbenzene (DVB) type with sulphonic groups.

In the event the aqueous solution is a fermentation broth bringing an aqueous solution to a pH value of between 1 and 4 may comprise fermenting a fungus in the fermentation broth. Preferably, fermenting a fungus in the fermentation broth comprises producing succinic acid in an amount sufficient to bring the pH of the fermention broth to a value of between 1 and 4. In the event bringing the fermentation broth to a pH of between 1 and 4 comprises fermenting a fungus, removing organic components from a fermentation broth is preferably carried out after said bringing.

In a preferred embodiment, the crystallizing of succinic acid in the process of the invention comprises a step of crystallizing the succinic acid from the aqueous solution to form succinic acid crystals and a mother liquor, wherein the aqueous solution is a fermentation broth. Preferably, the process for crystallizing succinic acid comprises recycling the mother liquor to the step of crystallizing the succinic acid. It was found that recycle of the mother liquor to the crystallizing step increased the yield of succinic acid crystals produced.

In the event the aqueous solution is a complex medium such as a fermentation broth or a mother liquor, the aqueous solution is usually treated with a cation exchange resin and / or an anion exchange resin.

Preferably, the process for crystallizing succinic acid comprises a step of washing the succinic acid crystals. Preferably, the process for crystallizing succinic acid comprises a step of drying the succinic acid crystals.

In a preferred embodiment, the process for crystallizing succinic acid further comprises a step of dissolving the succinic acid crystals from a step of crystallizing succinic acid from an aqueous solution, preferably a fermentation broth, to obtain a second aqueous solution comprising dissolved succinic acid, and recrystallizing the dissolved succinic acid to form succinic acid crystals.

A step of dissolving succinic acid crystals in a preferred process according to the present invention may be carried out at a temperature of between 30 and 90 degrees Celsius, preferably between 35 and 85 degrees Celsius, more preferably between 40 and 80 degrees Celsius. It was found advantageous that the dissolving of succinic acid crystals is carried out at a temperature of between 30 and 90 degrees Celsius, since a higher amount of succinic acid is dissolved in a lower amount of water as compared to dissolving succinic acid at a temperature of below 30 degrees Celsius. It was found advantageous to limit water consumption in the process according to the present invention, since this reduces the amount of energy and steam needed to evaporate water during subsequent crystallization of succinic acid from the solution comprising dissolved succinic acid in the process according the present invention.

In one embodiment an aqueous solution may be any solution in a crystallization process of the present invention. An aqueous solution may comprise dissolved succinic acid and/or succinic acid in crystal form. Preferably, the aqueous solution is a fermentation broth, a mother liquor, or a second aqueous solution, wherein the second aqueous solution preferably comprises dissolved succinic acid. Preferably the aqueous solution is a mother liquor.

In the event the aqueous solution in the process of the present invention is a fermentation broth, the process preferably comprises a step of removing organic components from the fermentation broth prior to treating the aqueous solution with an oxidizing agent. Organic components as used herein may be any insoluble and/or soluble organic components such as proteins and microbial cell (fragments). Removal of organic components from the fermentation broth may be carried out by any suitable method in the art, for instance filtration such as microfiltration, centrifugation, heat treatment, enzymatic treatment or any suitable combination of these methods.

In a preferred embodiment, the oxidizing agent in the process according to the present invention is ozone, peroxide, hypochlorite, permanganate, dichromate, or nitric acid. Preferably the oxidizing agent is ozone.

Treating an aqueous solution with an oxidizing agent in the process according to the present invention may be carried out in any suitable way. Preferably, the treating is carried out during a period of between 1 min and 3 h, preferably between 5 min and 2h more preferably between 10 min and 60 min. The period for treating an aqueous solution with an oxidizing agent depends on the amount of succinic acid and impurities present in the aqueous solution The concentration of oxidizing agent may vary between 0.1 to 10 g ozone / L aqueous solution, preferably between 0.2 and 8 g ozone / L aqueous solution. Preferably, the mass ratio of oxidizing agent to succinic acid is between 0.0001 to 0.8, preferably between 0.001 to 0.5, preferably between 0.002 to 0.2.

In a preferred embodiment the process according to the present invention further comprises fermenting a microbial cell which produces succinic acid in a suitable fermentation broth. The fermentation broth may be any suitable broth allowing growth of a microbial cell and production of succinic acid. The fermentation broth comprises any suitable carbon source such as glucose, fructose, galactose, xylose, arabinose, sucrose, lactose, raffinose and glycerol. Fermenting a microbial cell may be carried out under aerobic conditions, anaerobic conditions, micro-aerophilic or oxygen limited conditions, or a combination of these fermentation conditions, for instance as disclosed in WO2009/083756. An anaerobic fermentation process is herein defined as a fermentation process run in the absence of oxygen or in which substantially no oxygen is consumed, preferably less than 5, 2.5 or 1 mmol/L/h, and wherein organic molecules serve as both electron donor and electron acceptors.

The fermenting of a microbial cell may be carried out at any suitable pH between 1 and 9, depending on the microbial cell. In the event the microbial cell is a bacterial cell, the pH in the fermentation broth preferably is between 5 and 8, preferably between 5.5 and 7.5. Usually the pH of the bacterial fermentation broth is maintained at these values by adding neutralizing agents such potassium- or sodium hydroxide, or ammonium. In the event the microbial cell is a fungal cell the pH in the fermentation broth may range between 1 and 7, preferably between 2 and 6, preferably between 2.5 and 5. During fermentation of a fungal cell the pH usually decreases to a pH of between 1 and 4, preferably between 2 and 3. A suitable temperature at which the fementing of a microbial cell may be carried out in the process according to the present invention may be between 5 and 60 degrees Celsius, preferably between 10 and 50 degrees Celsius, more preferably between 15 and 40 degrees Celsius, more preferably between 20°C and 30 degrees Celsius, depending on the microbial cell. The skilled man in the art knows the optimal temperatures for fermenting a microbial cell in the process of the invention.

In one embodiment, the microbial cell is a bacterium from the genus *Mannheimia, Anaerobiospirillum, Bacillus,* or *Escherichia,* or a fungal cell from the genus *Saccharomyces, Aspergillus, Penicillium, Pichia, Kluyveromyces, Yarrowia, Candida, Hansenula, Humicola, Torulaspora, Trichosporon, Brettanomyces, Rhizopus, Zygosaccharomyces, Pachysolen or Yamadazyma.* Preferably, a bacterial cell belongs to a species *Mannheimia succiniciproducens, Anaerobiospirillum succiniciproducens Bacillus amylophylus, B. ruminucola* and *E. coli,* preferably an *E. coli.* Preferably, a fungal cell belongs to a species *Saccharomyces cervisiae, Saccharomyces uvarum, Saccharomyces bayanus, Aspergillus niger, Penicillium chrysogenum, P. symplissicum, Pichia stipidis, Kluyveromyces marxianus, K. lactis, K. thermotolerans, Yarrowia lipolytica, Candida sonorensis, C. glabrata, Hansenula polymorpha, Torulaspora delbrueckii, Brettanomyces bruxellensis, Rhizopus orizae* or *Zygosaccharomyces bailii.* Preferably, a fungal cell is a yeast, preferably a *Saccharomyces cerevisiae.*

The microbial cell according to the present invention may be any suitable wild-type organism, or a genetically modified microorganism. Suitable genetically modified *E. coli* cells are disclosed in Sanchez et al., Metabolic Engineering, 7 (2005) 229-239, WO2006/031424, and US 7,223,567. Suitable fungal cells are disclosed in WO2009/065780 and WO2009/065778.

In another preferred embodiment, the process according to the present invention is carried out on an industrial scale. Industrial scale as used herein is defined as crystallizing succinic acid in a volume of at least 100 litres, preferably at least 1 m$^3$, preferably at least 10 m$^3$, more preferably at least 100, or 1000 m$^3$, usually below 5000 m$^3$.

Succinic acid in crystal form obtained by the process according to the present invention may be used in any suitable application, such as the production of polymers.

In one embodiment the process according to the invention further comprises using the succinic acid in crystal form in the preparation of a polyester polymer, such as polybutylene succinate. It was found advantageous to use the succinic acid crystals obtained in the process according to the invention in the preparation of a polyester polymer, since this resulted in a high quality polymer with low coloration.

In one embodiment the present invention relates to the use of an oxidizing agent for removing impurities from an aqueous solution comprising succinic acid and crystallizing the succinic acid to form succinic acid crystals.

**FIGURES**

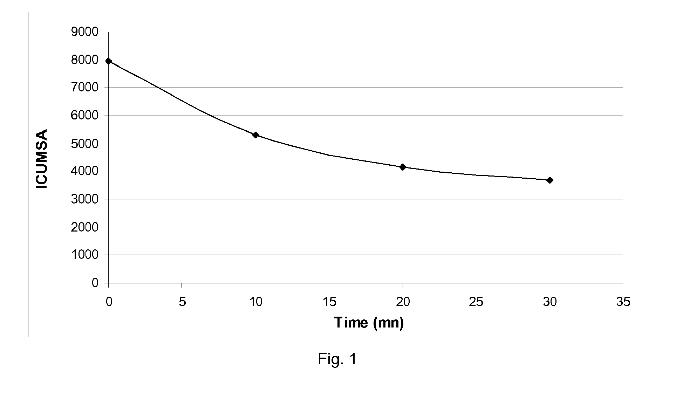

**Figure 1**. Effect of ozone treatment on coloration of mother liquor.

**EXAMPLES**

**GENERAL MATERIALS AND METHODS**

**Color measurement**

The color in solution is measured at 420 nm in a spectrophotometer UVIKON 923 from Bio-Tek KONTRON. Coloration is expressed in ICUMSA unit (International Commission for Uniform Methods of Sugar Analysis), which was calculated as:

$$ICUMSA = (Abs \: 420_{nm} \times 100000) / Brix$$

1° Brix is equal to 1% (w/w) of saccharose in solution determined in a refractometer.

## Determination sulphur and cations

Sulphur and cations were determined by atomic emission spectrometry using inductively coupled plasma.

## Determination anions

Anions such as sulfate, chloride and phosphate were separated on an anion exchange column, Dionex AS11-HC type, which was heated at 36 degrees Celsius, and detected by conductimetry. The eluent was a gradient of progressively increasing NaOH concentration. Trifluoroacetic acid was used as internal standard.

## Determination organic acids

Organic acids were separated with ion exchange chromatography on columns (3 in series) from Biorad HPX-87H type, which were heated at 85 degrees Celsius and detected with UV at 210 nm. The elution solvent was a 5 mM sulphuric acid solution. The quantitative analysis was performed by external calibration. Pyruvic, malic, fumaric, lactic, formic and acetic acids were used as standards. The chromatographic separation was conducted in isocratic mode 60 microliters of a 3% succinic acid solution were injected.

## Example 1: Succinic acid production by *E. coli* and recovery of succinic acid from

## fermentation broth

### 1.1 E.coli fermentation

The strain *Escherichia coli* SBS550MG — pHL413 (genotype $\Delta adhE \Delta ldhA \Delta iclR \Delta ackpta$, PYC), was prepared as disclosed in US 7,223,567, which is herein included by reference. The fermentative production succinic acid consisted of growth phase, transition phase and production phase.

### Growth phase

*Escherichia coli* strain SBS550MG — pHL413 was inoculated in two 500 ml shake flasks with LB medium (Trypton 10 g/L, Yeast extract 5 g/L, NaCl 10 g/L, Ampicillin, carbenicillin, oxacillin 67 mg/L each), which were incubated at 37°C, for 24h, until an OD of 7 was reached. Subsequently, the contents of the shake-flasks were transferred to a 1 $m^3$ fermenter, which contained 700 L of the following medium: $K_2HPO_4$ 3.6; $MgSO_4.7H_2O$ 1 g/l; citric acid 2 g/l; glucose 5 g/l; $ZnSO_4.7H_2O$ 30 mg/l; $CuCl_2.2H_2O$ 10 mg/l; $MnSO_4.H_2O$ 30 mg/l; $CoCl_2.6H_2O$ 1.2 mg/l; $H_3BO_3$ 1 mg/l; $Na_2MoO_4.2H_2O$ 6 mg/l; $Al_2(SO_4)_3.18H_2O$ 7.5 mg/l ; $NiSO_4.6H_2O$ 4 mg/l ; $FeSO_4.7H_2O$ 90 mg/l; $CaCl_2.2H_2O$ 300 mg/l; Biotine 1 mg/l; Thiamine 1 mg/l; Ampicilline 200 mg/l.

The pH and temperature were controlled at 6.75 by addition of 25 wt% ammonia and at 37 degrees Celsius, respectively. An airflowrate of 700 L/min was applied (1 vvm). The dissolved oxygen level ($pO_2$) was controlled at >20% by adapting the stirrer speed. After consumption of initial 5 g/L, glucose concentration was kept limited by controlled feed of a 50 wt% solution to the fermenter. An exponential feed rate was applied. After 27h and consumption of 25 g/L (referring to post inoculum volume) fed glucose, 10 g/L of biomass was produced (OD 30).

### Transition phase

Subsequently, a phase at zero oxygen pressure ($pO_2$) was applied during 6.5 h by manually controlled stirring, at pH 5 by controlling with 5 N NaOH and glucose feed at 1.6 g/L/h (refers to post inoculum volume).

### Production phase

The succinic acid production phase was started by switching to 0.1 vvm $CO_2$ supply at 0.1 vvm and adding 75 g/L of glucose (ref post inoc volume). pH and temperature were controlled at 6.75 and 37 degrees Celsius, respectively .

After 93h of fermentation, 45 g/L of succinic acid was obtained.

1.2. Recovery of succinic acid from fermentation broth

The fermentation broth was filtered by tangential filtration with tangential flow on a ceramic membrane with a channel diameter of 3.5 mm, having a pore diameter of 100 nm, with a trans-membrane pressure of 1 bar resulting in a mean flow of 90 l/h/m$^2$. The temperature was maintained at 60°C. Subsequently, the fermentation broth was heated to 80°C for 10-15 min, which allowed flocculation of proteins. The solution was subsequently filtered through a filter with a pore diameter of 0.22 $\mu$m.

The solution was then treated with a weak cation exchange resin, Amberlite IRC747 and supplied at a flow rate of 2 Bed Volumes (BV)/h at 60°C. The cation exchange treatment reduced the amount of divalent ions (Ca$^{2+}$ and Mg$^{2+}$) to below 5 ppm.

The solution obtained after treatment with cation exchange resin was treated with water-dissociation bipolar electrodialysis, EUR6 from Eurodia. The flow of cations was approximately 22 eq/h/m$^2$, converting 90% of succinate to succinic acid. The pH at the end of the electrodialysis was about 3.5. For reasons of energy saving, the degree of conversion was fixed at 90%.

The solution obtained after water-dissocation bipolar electrodialysis, was treated with a strong cationic resin (Purolite C150). The cationic resin treatment was done at 40°C and at 2 BV/h. The volume of the solution treated was approximately 10-15 bed volumes. The pH of the solution after treatment on cationic resin was 2.

The acidified solution was concentrated by evaporation of water on a Wiegand® falling-film evaporator at 80 degrees Celsius, until supersaturation was reached (approximately 420 g/l).

Subsequently, the solution was cooled at a rate of 5 degrees Celsius/h from 80 to 20 degrees Celsius and succinic acid was crystallized. The succinic acid crystals were separated in a ROUSSELET® centrifuge and the remaining mother liquor was used in for treatment with ozone as described in Example 2.

After washing with one volume of demineralized water per cake volume, the succinic acid crystals obtained were dried, resulting in a yield of succinic acid of > 85% with a purity of 99.7%.

**Example 2. Treatment of a succinic acid containing mother liquor with ozone**

2.1. Treatment of mother liquor

A mother liquor obtained in Example 1 was treated with ozone in batch mode. 50L of mother liquor was introduced in a buffer tank connected to a CFS06 OZONIA reactor. A flow rate of ozone (O$_3$) was fixed at 450 g/h, corresponding to a mass ratio (O$_3$/SA) of 0.075. Samples were taken for analysis and colour measurement (ICUMSA) every 10 min for 30 min.

Ozone treatment showed a rapid decrease in coloration during the first 20 min (Figure 1). The residual coloration after 30 min of the mother liquor was about 46% (3800 IU of ICUMSA) of the original coloration.

This shows that recycle of mother liquor to the (acidified) solution from which succinic acid crystals were crystallized as described in Example 1 would be possible, without affecting the color thereof. Furthermore, ozone treatment of the mother liquor showed a decrease in fumaric and orotic content after treatment, as illustrated in Table 1.

**Table 1. Content of impurities in mother liquor containing succinic acid before and after treatment with ozone during 30 min**

|  | Mother liquor before treatment | Mother liquor after 30 min ozone treatment |
|---|---|---|
| Succinic acid (%) | 61.6 | 64.3 |
| Compound | w/w ratio: (weight compound/weight succinic acid) | w/w ratio: (weight compound/weight succinic acid) |
| Lactic acid | ∼ 308.4 x10$^{-3}$ | ∼ 295.5 x10$^{-3}$ |
| Formic acid | 39.1 x10$^{-3}$ | 44.2 x10$^{-3}$ |
| Acetic acid | 21.9 x10$^{-3}$ | 26.0 x10$^{-3}$ |
| Fumaric acid | 1.8 x10$^{-3}$ | 0.5 x10$^{-3}$ |
| Pyruvic acid | ∼ 13.0 x10$^{-3}$ | ∼ 9.3x10$^{-3}$ |
| Malic acid | ∼ 48.7 x10$^{-3}$ | ∼ 46.7 x10$^{-3}$ |

(continued)

| | Mother liquor before treatment | Mother liquor after 30 min ozone treatment |
|---|---|---|
| Orotic acid | $50.3 \times 10^{-3}$ | $35.8 \times 10^{-3}$ |
| Chlorine acid | $16.2 \times 10^{-3}$ | $15.6 \times 10^{-3}$ |
| Sulfate | $17.9 \times 10^{-3}$ | $18.7 \times 10^{-3}$ |
| Phosphate | $211.0 \times 10^{-3}$ | $202.2 \times 10^{-3}$ |
| Thiosulfate | $< 1.6 \times 10^{-3}$ | $< 1.6 \times 10^{-3}$ |
| Citrate | $795.5 \times 10^{-3}$ | $948.7 \times 10^{-3}$ |

2.2. Crystallization of succinic acid from mother liquor

Both a mother liquor that was treated with ozone as described in Example2.1. and a mother liquor that was not treated with ozone, were concentrated by evaporation at 80 degrees Celsius until succinic acid supersaturation was reached (420 g/l) in a rotary vacuum system (Rotavapor R-215). Subsequently, the supersaturated solutions were cooled at a rate of 5 degrees Celsius/h from 80 to 20 degrees Celsius in a stirred 2L reactor. The formed succinic acid crystals were separated with a Buchner and washed with 1 volume of cold demineralised water / volume of cake. The succinic acid crystals were dried in an oven (60°C) under vacuum.
Table 2 shows that ozone treatment of the mother liquor resulted in succinic acid crystals with a decreased of fumaric and orotic acid content.

Table 2 : Impurities of succinic acid crystals obtained from mother liquor before and after treatment with ozone

| | Succinic acid cystals from mother liquor before treatment | Succinic acid cystals from mother liquor after treatment |
|---|---|---|
| Succinic acid (%) | 97.4 | 98.2 |
| Compound | w/w ratio: (weight compound/weight succinic acid) | w/w ratio (weight compound/weight succinic acid) |
| Lactic acid | $< 5.13 \times 10^{-3}$ | $< 5.09 \times 10^{-3}$ |
| Formic acid | $< 1.03 \times 10^{-3}$ | $< 1.02 \times 10^{-3}$ |
| Acetic acid | $< 1.03 \times 10^{-3}$ | $< 1.02 \times 10^{-3}$ |
| Fumaric acid | $1.75 \times 10^{-3}$ | $0.71 \times 10^{-3}$ |
| Pyruvic acid | $< 0.21 \times 10^{-3}$ | $< 0.20 \times 10^{-3}$ |
| Malic acid | $< 1.03 \times 10^{-3}$ | $< 1.02 \times 10^{-3}$ |
| Orotic acid | $8.42 \times 10^{-3}$ | $2.85 \times 10^{-3}$ |
| Chlorine acid | $< 0.10 \times 10^{-3}$ | $< 0.10 \times 10^{-3}$ |
| Sulfate acid | $< 0.10 \times 10^{-3}$ | $< 0.10 \times 10^{-3}$ |
| Phosphate acid | $0.72 \times 10^{-3}$ | $0.51 \times 10^{-3}$ |
| Thiosulfate acid | $< 0.10 \times 10^{-3}$ | $< 0.10 \times 10^{-3}$ |
| Citrate | $2.36 \times 10^{-3}$ | $1.93 \times 10^{-3}$ |

**Claims**

1.  Process for crystallizing succinic acid from an aqueous solution comprising treating the aqueous solution with an oxidizing agent and crystallizing the succinic acid to form succinic acid crystals.

**2.** Process according to claim 1, **characterized in that** the crystallizing of succinic acid comprises a step of crystallizing the succinic acid from the aqueous solution to form succinic acid crystals and a mother liquor, wherein the aqueous solution is a fermentation broth.

**3.** Process according to claim 2, further comprising recycling the mother liquor to the step of crystallizing the succinic acid.

**4.** Process according to claim 2 or 3, further comprising a step of dissolving the succinic acid crystals from the step of crystallizing succinic acid to obtain a second aqueous solution comprising dissolved succinic acid, and recrystallizing the dissolved succinic acid to form succinic acid crystals.

**5.** Process according to any one of the claims 1 to 4, **characterized in that** the aqueous solution is a fermentation broth, a mother liquor, and/or a second aqueous solution comprising dissolved succinic acid.

**6.** Process according to any one of the claims 1 to 5, **characterized in that** the oxidizing agent is ozone or peroxide, hypochlorite, permanganate, dichromate, or nitric acid.

**7.** Process according to any one of the claims 2 to 6, further comprising fermenting a microbial cell which produces succinic acid in a suitable fermentation broth.

**8.** Process according to any one of the claims 1 to 7 further comprising using the succinic acid in crystal form in the preparation of a polyester polymer

**9.** Use of an oxidizing agent for removing impurities from an aqueous solution comprising succinic acid and crystallizing the succinic acid to form succinic acid crystals.

Fig. 1

Europäisches
Patentamt
European
Patent Office
Office européen
des brevets

# EUROPEAN SEARCH REPORT

Application Number

EP 10 15 8468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 1 945 175 A (BERTSCH JOHANN A ET AL) 30 January 1934 (1934-01-30) * the whole document * ----- | 1-9 | INV. C07C51/43 C07C51/487 C07C55/10 |
| X | US 3 338 959 A (THOMAS SCIANCE CARROLL ET AL) 29 August 1967 (1967-08-29) * claim 1 * ----- | 1-9 | |
| X | GB 1 366 933 A (ICI LTD) 18 September 1974 (1974-09-18) * the whole document * ----- | 1-9 | |

TECHNICAL FIELDS
SEARCHED       (IPC)

C07C

The present search report has been drawn up for all claims

| Place of search | Date of completion of the search | Examiner |
|---|---|---|
| Munich | 22 July 2010 | Cooper, Simon |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 15 8468

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2010

| Patent document cited in search report | | Publication date | Patent family member(s) | Publication date |
|---|---|---|---|---|
| US 1945175 | A | 30-01-1934 | NONE | |
| US 3338959 | A | 29-08-1967 | NONE | |
| GB 1366933 | A | 18-09-1974 | NONE | |

For more details about this annex : see Official Journal of the European Patent Office, No. 12/82

## REFERENCES CITED IN THE DESCRIPTION

**Patent documents cited in the description**

- US 5168055 A
- US 6265190 B
- WO 2009082050 A
- WO 2009083756 A
- WO 2006031424 A
- US 7223567 B
- WO 2009065780 A
- WO 2009065778 A

**Non-patent literature cited in the description**

- **SANCHEZ et al.** *Metabolic Engineering,* 2005, vol. 7, 229-239